(19) **Europäisches Patentamt / European Patent Office / Office européen des brevets**

(11) **EP 1 974 662 B1**

(12) **EUROPEAN PATENT SPECIFICATION**

(45) Date of publication and mention
of the grant of the patent:
**21.05.2014 Bulletin 2014/21**

(51) Int Cl.:
*A61B 5/024* *(2006.01)* *A61B 5/11* *(2006.01)*
*A61B 5/0205* *(2006.01)* *A61B 5/021* *(2006.01)*

(21) Application number: **07105043.9**

(22) Date of filing: **27.03.2007**

(54) **Fall detector**

Falldetektor

Détecteur de chute

(84) Designated Contracting States:
**CH DE FR LI**

(43) Date of publication of application:
**01.10.2008 Bulletin 2008/40**

(73) Proprietor: **CSEM**
**Centre Suisse d'Electronique et de**
**Microtechnique SA**
**2002 Neuchâtel (CH)**

(72) Inventors:
• **Bertschi, Mattia**
**1093, La Conversion (CH)**
• **Vetter, Rolf**
**1116, Cottens (CH)**

(74) Representative: **GLN SA**
**Avenue Edouard-Dubois 20**
**2000 Neuchâtel (CH)**

(56) References cited:
**EP-A1- 1 366 712**     **WO-A-2004/100092**
**WO-A1-2006/074253**     **US-A- 6 160 478**
**US-A1- 2003 153 836**     **US-A1- 2005 177 929**
**US-B1- 6 397 151**

• **DEGEN T ET AL: "SPEEDY:a fall detector in a wrist watch" WEARABLE COMPUTERS, 2003. PROCEEDINGS. SEVENTH IEEE INTERNATIONAL SYMPOSIUM ON 21-23 OCT. 2003, PISCATAWAY, NJ, USA,IEEE, 21 October 2003 (2003-10-21), pages 184-187, XP010673821 ISBN: 0-7695-2034-0**
• **CHEN ET AL: "Wearable Sensors for Reliable Fall Detection" ENGINEERING IN MEDICINE AND BIOLOGY SOCIETY, 2005. IEEE-EMBS 2005. 27TH ANNUAL INTERNATIONAL CONFERENCE OF THE SHANGHAI, CHINA 01-04 SEPT. 2005, PISCATAWAY, NJ, USA,IEEE, 2005, pages 3551-3554, XP031001295 ISBN: 0-7803-8741-4**

**Description**

Technical field

The present invention concerns the field of the safety of people, in particular of the elderly. It relates, in particular, to a fall detection method and to a fall detector implementing this method.

Background of the invention

Falls are the most widespread domestic accidents among the elderly and their consequences often give rise to impairments to the health and lifestyle of the victims. Of course, physical after-effects and other injuries are the direct consequences of these accidents and it is possible to associate significant costs with them, related to the medical treatments incurred. By way of example, it has been reported that, in the year 2000, in the United States, fatal falls and nonfatal falls cost 0.2 billion and 19 billion dollars respectively.

Furthermore, it frequently happens that elderly people who have fallen a first time then fear a new fall and sink gradually into inactivity and social isolation. The reduction in their mobility leads progressively to an increase in the risk of a fall. Given the growing part played by the elderly in our modern societies, the socio-economic impact that this self-imposed isolation could have cannot be neglected.

The most widespread solution, if not for avoiding falls, then at least for limiting apprehension of a fall, is afforded by fall detectors which consist of portable devices furnished with a button for triggering an alarm and with telecommunication means suitable for transmitting this alarm. However, after a fall, the person may not be in a state to actuate the button and to trigger the alarm (unconsciousness, state of shock, broken arm, etc.).

To alleviate this drawback, autonomous fall detectors have been developed, capable of triggering an alarm automatically without any intervention of the victim. These autonomous detectors operate essentially on two principles. The detector can be sensitive to the person's appearance or impact on its environment and comprise video or vibratory type sensors placed in the usual surroundings of the subject. The benefit of these devices is that they do not have to be worn. Instead, they are fixed in a given spot and cannot be moved when the person changes location. Furthermore, depending on the environment, finding the appropriate settings in order to pick up vibrations properly may turn out to be complex. In the case of a video sensor, the person can sometimes have the impression of being monitored and be inconvenienced thereby. The detector can also be worn by the person and detect a fall directly when it occurs, triggering an alarm immediately. In this case, information provided by inclinometers, gyroscopes or accelerometers is exploited. These devices are generally compact, inexpensive, fairly non-intrusive and easy to use. They can be worn in various places on the body (chest, pelvis, wrist, knee, etc.), which may also make it tricky to find the appropriate settings.

For the detectors of this second type, sensors worn in proximity to the centre of gravity are those which give the best results. Thus, the device described in patent application FR 2 829 862, which is placed on the trunk of the person, is known. It detects an abrupt movement along a first axis and, should such a movement be detected, measures the inclination of the body so as to determine whether or not the person is prostrate. These measurements are conditioned on a first detection of vital vibrations, so as to perform the subsequent measurements only if the device is being worn, to save power. The possible triggering of an alarm then follows a quantitative approach, that is to say it is triggered if the values measured for vital vibrations, movement along the first axis, and inclination are above or below a predetermined threshold.

However, the devices situated in proximity to the centre of gravity are those which are the least pleasant to wear on a daily basis, in particular in respect of commonplace actions, such as going to the toilet, getting dressed or sleeping, etc..

It would seem that a device having substantially the form of a wristwatch would be well tolerated in all situations. But the changes of position and the accelerations that the wrist experiences during everyday actions make it very difficult to detect a fall. The latter is poorly differentiated from a trivial impact like a hand striking a table. Furthermore, the method proposed in the patent application cited above cannot be adapted to a device worn on the wrist, in particular since measurement of the inclination of the forearm cannot give reliable information about the person's position. Generally, fall detectors placed on the wrist give rise to a large number of false alerts, and this may generate significant and unnecessary costs. US 2003/0153836 discloses a fall-detector which is worn on the wrist.

The present invention is aimed at proposing a method for detecting a fall of a person and a device implementing this method that can be worn by a person, in particular on the wrist, without causing inconvenience and offering excellent reliability in the detection of a fall.

Disclosure of the invention

More precisely, the invention relates to a method of detecting a fall of a person as disclosed by claim 1.

The invention also relates to fall detectors worn on the wrist of a person, comprising a memory, an accelerometric sensor and, possibly a sensor or several sensors providing information about the cardiovascular system, means for transmitting an alarm and a microprocessor designed to perform one of the methods above.

Brief description of the drawings

Other details will be more clearly apparent on reading the following description, given as a non-limiting example, with reference to the appended drawing, in which:

- Figure 1 is a diagram illustrating the various steps of the detection of a fall,
- Figure 2a shows the reference signature in terms of mean and standard deviation of a large number of diverse falls, suffered by several subjects, and
- Figure 2b shows the signature obtained for a trivial striking of the hand on a table by a person wearing a fall detector according to the invention.

The invention which will now be described relates to a method for detecting a fall of a person equipped with a suitable device worn on the wrist. Advantageously, so as not to inconvenience them in their daily actions, the device can take the form of a wristwatch.

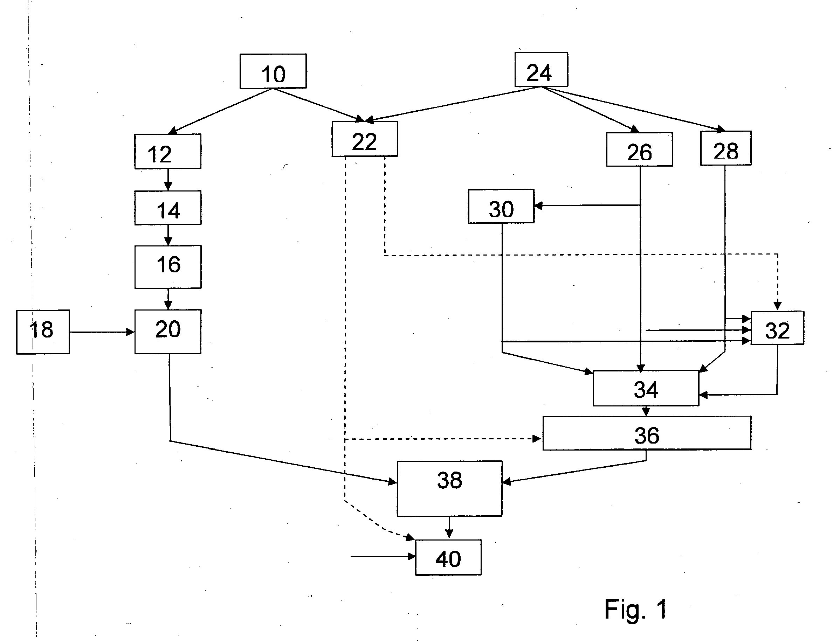

The device comprises a sensor 10 providing an accelerometric signal making it possible to obtain an approximation of the person's kinetic energy in the three dimensional space. Such a sensor can be an accelerometer or a gyroscope. The sensor provides an item of information from which the kinetic energy of the body is estimated via the quadratic norm of multidimensional signals obtained via the sensor. The person skilled in the art can also combine the information from several of these sensors. Furthermore, the device comprises a memory and a microprocessor programmed to perform the operations which will now be detailed, with reference to Figure 1.

If the sensor 10 is an accelerometer, the latter measures, at a determined frequency, typically from 100 to 900 Hz, the acceleration experienced at the wrist of the person wearing the device. The microprocessor is programmed to extract, at 12, from the three dimensional accelerometer signals, a signature $S_t$ at an instant t, representing the distribution over a time window $\Delta t$ including the instant t, of the kinetic energy of the person. This distribution is approximated by the quadratic norm in the present application:

$$e(t) = a_x^2(t) + a_y^2(t) + a_z^2(t)$$

where $a_x$, $a_y$ and $a_z$ represent the acceleration in the respective directions.

It has in fact been noted that this distribution of the kinetic energy over a time window $\Delta t$ provides a particularly reliable signature, that is to say it makes it possible to identify a given event happening to the person. Thus, if a signature recorded during a simulation of a fall is recognized during a real situation, this indicates with a high probability that a fall has actually occurred.

Thus, in a particularly advantageous manner, fall detection can be based on a qualitative approach, namely a similarity measure between a reference signature and the evolution of the kinetic energy over a time window $\Delta t$ rather than just on a quantitative measurement, that is to say a comparison of the instantaneous kinetic energy relative to a predefined threshold value.

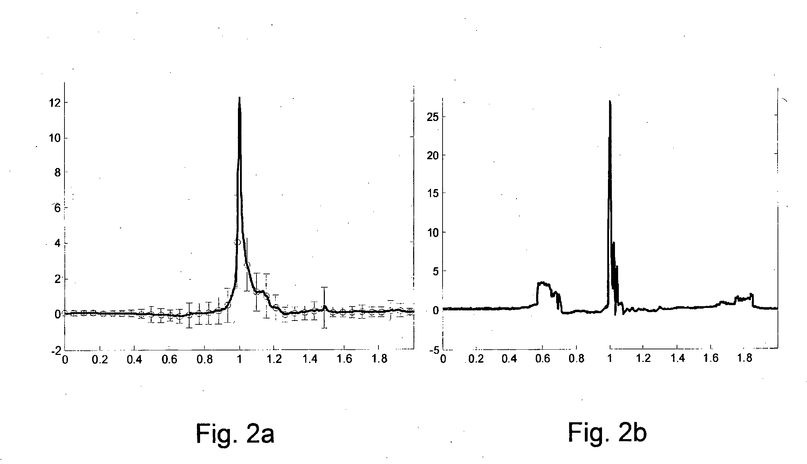

To illustrate this, we shall refer to Figure 2, which shows, at a, the reference signature in terms of means and standard deviations of a large number of diverse falls, suffered by several subjects, according to the predefined procedure. Represented at b is a signature obtained when the hand of the person simply hits a table. It is indeed seen that, merely using a quantitative approach, an alert would be triggered in both cases, while, qualitatively, that is to say by measuring the resemblance between the cumulative distances of all the samples of both signatures, it is possible not to respond with an alarm to the situation of Figure 2b.

Thus, the detection of a fall therefore relies mainly on a qualitative comparison of the signature $S_t$ with reference values recorded in the memory in terms of means and variances of the reference signature. To obtain them, one records the signatures $S_t$ representing the distribution over a time window $\Delta t$ of the kinetic energy of a mannequin or preferably of one or more human guinea pigs, by simulating all sorts of falls, in all directions in space. Preferably, human guinea pigs with neuromuscular reflexes representative of the elderly will be used. As and when real falls of elderly people are recorded, their signatures can advantageously be substituted for the simulated fall signatures.

To obtain the reference signature $S_{ref}$, the plurality of signatures recorded in a database is used to obtain statistics in the form of means and variances of a reference signature as follows. A particular value of each signature is identified so as to carry out an alignment, for example a maximum, a minimum, a zero crossing, a maximum slope, etc.

This particular value is used to align the signatures, so as to obtain a group of aligned fall signatures. On the basis of the various aligned signatures, the reference signature $S_{ref}$ is obtained by averaging, at each instant t, the values of the aligned signatures. The squared value of the acceleration due to gravity is then deducted so as to remove its influence and the signature $S_{ref}$ is normalised. For each instant t, the variance of the values of the aligned signatures is also determined. These variance values are placed on the diagonal of a matrix $\theta$. As will be seen below, $S_{ref}$ and $\theta$ are used to compare the instantaneous signature St with the reference signature $S_{ref}$.

The instantaneous signature $S_t$, at a given instant t, is extracted by the microprocessor at 12. St represents the distribution of the kinetic energy measured by the sensor over a time window $\Delta t$ typically of the order of 2 to 3 seconds. The instantaneous signature is then, at 14, normalised by an infinity or second order norm so as to be compared with $S_{ref}$. Next, at 16, a particular value of the signature $S_t$ is identified, for example a maximum, a minimum, a zero crossing or a maximum slope, etc. The signature $S_t$ is shifted so as to bring the particular value to the middle of the time window $\Delta t$. It will be noted that the particular value will be the same as that which was chosen for the alignment of the signatures recorded during the determination of the reference signature $S_{ref}$. In this way, given that a signature St is obtained at an instant t which is arbitrary with respect to the course of a possible fall, the particular value mentioned above is used to align, over time, the signature $S_t$ and the reference signature $S_{ref}$, so that they can be compared.

At a predetermined frequency, which can be between once a second and once every 10 seconds, roughly, the signature $S_t$ is compared with the reference signature $S_{ref}$, in the block 20.

The microprocessor is programmed so as to compare qualitatively these two signatures, by means of similarity measurements. This operation amounts to determining the similarity between $S_t$ and the reference signature Sref by comparing the samples forming each of the signatures. Several mathematical procedures can be used for this, in particular ones based on distance calculations.

Among the large number of samples that the reference signature $S_{ref}$ comprises, the reliability of some is not good, since the variances estimated over all the reference signatures are too big. In order to minimize the influence of these overly atypical values, it is proposed to use, according to an advantageous aspect of the invention, the Mahalanobis distance:

$$d(S_t, S_{ref}) = e^{-(S_t - S_{ref})^T \theta^{-1}(S_t - S_{ref})}$$

The way in which $S_{ref}$ and the matrix $\theta$ were obtained was described above. The variances make it possible to lower the influence of the least reliable samples. The Mahalanobis distance in its above form is normalised, i.e. it can take values lying between zero and one. It therefore gives directly a magnitude representative of the probability that the subject has fallen. Other details regarding the application of the Mahalanobis distance and normalisation methods can be found in the book by J.R. Deller, J.G. Proakis, and J.H.L. Hansen, "Discrete-Time Processing of Speech Signals", Prentice Hall, New Jersey 07458, 1987.

Depending on the sensitivity desired, a threshold can be defined by the person skilled in the art, above which the probability that a fall has occurred, approximated by the Mahalanobis distance above, requires the triggering of an alarm signal. The microprocessor analyses at 38 the result of the probability of a fall obtained by comparing $S_t$ and $S_{ref}$ for kinetic energy and possibly triggers at 40 a signal which gives rise to emergency treatment to assist the person.

The comparison between $S_t$ and $S_{ref}$ can be made regularly, according to a predetermined time interval. Preferably, this interval is chosen in correlation with the amplitude of the time window $\Delta t$, so as to guarantee that it is not possible for a fall not to be detected. It is indeed possible to estimate that the energy experienced by the wrist of a victim of a fall is disturbed for about one second. The time window is therefore determined in such a way that, considering the frequency at which the comparison step is performed, the maximum gap between two samples taken into account during a comparison is one second.

It is also possible to perform a comparison only when there is a potential risk that a fall has occurred, for example, when the measured energy exceeds a predetermined threshold.

Although the above description makes reference to an accelerometer, the signature can also be obtained using other sensors, such as a gyroscope.

According to the invention, the device comprises, in order to improve the reliability of fall detection, at least one or several sensors 24 for obtaining a cardiovascular parameter of the person. It has been shown that it is possible, in certain cases, to establish a relation between a fall and certain cardiovascular parameters of the person. Thus, cardiovascular-related disturbances can be the cause of a fall or result from such an accident. For example, losses of consciousness often arise from a fall in blood pressure to which the cardiovascular system of an elderly person reacts in a haphazard manner. In these cases of a fall, a drop in heart rate is also often observed.

Furthermore, a fall generally gives rise to disturbances in respect of the cardiovascular parameters. Thus, while it is normal to observe a drop in heart rate between the standing position and the lying position due to a decrease in the orthostatic pressure, a fall creates emotional stress which, even if the person is in supine position after the fall, gives rise to an increase in heart rate and blood pressure. This phenomenon is accentuated in the event of injury caused by the fall. Thus, detection of abnormal cardiovascular parameters, if said detection is accompanied by values provided by the previously mentioned accelerometric sensor showing the probability of a fall, strengthens the probability that such an event has occurred and enables the performance of the detector to be greatly improved.

The cardiovascular parameters can be obtained by a photoplethysmographic sensor which provides signals on the basis of which it is possible to estimate the heart rate, its variability and an item of information correlated with the blood pressure, making it possible to estimate whether said pressure is increasing or decreasing. The sensor can be placed in the back of the device, when the latter is in the form of a wristwatch. It is also possible for additional sensors to be placed on the fingers, communicating with the device. Though it is easy to determine heart rate on the basis of these signals, it is, on the other hand, trickier to obtain values for the blood pressure, since the signals provide only information on the variations in blood volume in the tissues, which are only partially correlated with the blood pressure. The probity of this estimation is therefore, preferably, limited by reference values, even after normalisation. Thus, as previously, the use of the values obtained as a reference is weighted by the variance.

The microprocessor is therefore programmed to extract, from the signals provided by the cardiovascular sensor, either the heart rate alone, at 26, or the blood pressure alone, at 28, or both of these items of information and the variability of the heart rate at 30.

The signals provided by the accelerometer also enable the microprocessor to evaluate, at 22, the person's level of activity. If the microprocessor estimates that the person is in a normal situation at the instant t, that is to say with no significant and abnormal variation in the accelerometric signals, the cardiovascular values extracted at each beat and oversampled in a regular manner at a frequency of 4Hz are stored in the memory, at 32, so as to serve subsequently as reference.

Therefore, the microprocessor is programmed to determine, for each of the cardiovascular parameters, a mean and a variance, over a determined period which has just elapsed. These reference values are therefore refreshed continuously so long as the activity indicator is low.

Thereafter, when the kinetic energy indicates that the probability of a fall is high, new cardiovascular parameters are extracted and normalised using the reference values recorded in the memory. At 34, the parameters obtained at the instant t are compared with the reference values stored in memory, by means of dissimilarity measurements. As previously, this comparison is done by determining a distance, for example the Mahalanobis distance.

$$d(S_{t,cardio}, S_{ref,cardio}) = 1 - e^{-(S_{t,cardio} - S_{ref,cardio})^T \theta_{cardio}^{-1}(S_{t,cardio} - S_{ref,cardio})}$$

$S_{t,cardio}$ is the instantaneous signature comprising one, two or three cardiovascular parameters as the case may be. $S_{ref,cardio}$ is the reference signature comprising the mean over time of one, two or three items of cardiovascular information. $\theta_{cardio}$ is the matrix of the variances of the cardiovascular information, taken over time.

For the signals provided by the cardiovascular sensor or sensors, a distance of 0 corresponds to normal signals, while a distance of 1 corresponds to signals that are theoretically most improbable for this state of activity. Stated otherwise, a distance of close to 1 indicates a probability that the cardiovascular signals are altered following a fall.

The microprocessor is then programmed to sum with an appropriate weighting, during step 38; the distance obtained with the cardiovascular signals with that obtained with the accelerometric signals. The weighting factors can be dynamically adjusted and are typically determined in function of the reliability of the respective distance measurements. It determines the probability of a fall by applying the rules given below.

If the accelerometric signals do not show any activity: it is probable that the person is static, in a sitting, standing or lying position. In this case, if the cardiovascular parameters show a too large blood pressure and heart rate, it is very likely that the person is in an emergency situation. On the other hand, if the cardiovascular parameters are normal, the likelihood of a fall is greatly reduced.

If the accelerometric signals show abnormal or non coordinated activity, it is probable that the person is in an emergency situation if the cardiovascular parameters show a too large blood pressure or heart rate. If the cardiovascular parameters are normal with respect to the situation measured by the accelerometric signals, the likelihood that a fall has occurred decreases significantly and it is not necessary to trigger an alarm.

If the accelerometric and cardiovascular signals show similar behaviours before and after an event potentially identified as a fall, then the likelihood that the event in question was not a fall or, anyway, that there is no need to trigger an alarm.

As an option, the microprocessor is, furthermore, programmed to determine, as a function of the cardiovascular parameters and/or of the accelerometric data that it receives, whether the device is or is not being worn. If it is not being worn, the microprocessor can warn the user or, directly, alert a call centre which will then take the necessary measures and try to contact the person or trigger an intervention.

Furthermore, the microprocessor can also be programmed to detect, on the basis of the cardiovascular signals noted, whether the sensor is not properly positioned. As above, the microprocessor can then alert the wearer or a call centre. A safety step 36 can be added to the data processing scheme. The cardiovascular parameters are considered to be unreliable and they are disregarded during step 38.

It is also possible to design a button for manual triggering of the alarm, as is commonplace in prior art devices.

Thus, a fall detection device is proposed that can be designed in a modular manner, that is to say it is possible to use an accelerometric sensor alone or to combine it with the cardiovascular sensor. The results obtained, particularly when the two sensors are combined, do not show any significant difference from a fall detector worn near the centre of gravity of the person. The reliability obtained is 91 % as against 65% for prior art devices worn on the wrist (see T. Degen, H. Jaeckal, M. Rufer, and S. Wyss, "SPEEDY: a fall detector in a wristwatch", Proc. On Wearable Computers, pp. 184-187, 2005).

**Claims**

1. Method of detecting a fall of a person, comprising the following steps:

    - obtaining a signature St at an instant t, representing the distribution over a time window Δt of the kinetic energy of the person, measured in three dimensions by an accelerometric sensor placed in a device located on the wrist of a user,
    - normalising said signature by an infinity or second order norm,
    - obtaining a normalized similarity measure based on a normalized distance between the normalized signature $S_t$ and the normalized reference signature $S_{ref}$, said similarity measure weighting samples of $S_{ref}$ and $S_t$ in function of their reliability, where the normalized reference signature $S_{ref}$ were obtained of a representative sample of falls,
    - obtaining at least one cardiovascular parameter of the person via a photoplethysmographic sensor placed in said device and providing photoplethysmographic signals,
    - using information from the accelerometric sensor used to detect fall to enhance the quality of the photoplethysmographic signals,
    - obtaining from the accelerometric sensor an item of information relating to the level of activity of the person and obtaining from the photoplethysmographic signals a reference signature $S_{ref,cardio}$ of the cardiovascular parameter for a given activity level,
    - obtaining a cardiovascular based comparison by means of dissimilarity measurements, based on a normalized distance between an instantaneous signature $S_{t,cardio}$ and the reference signature $S_{ref,cardio}$, and
    - combining the results of the accelerometer and cardiovascular based comparisons by summing with an appropriate weighting, the distance obtained with the photoplethysmographic signals with that obtained with the accelerometric signals,
    - using the result of the combination as an indicator of probability of fall, and
    - possibly, triggering a warning signal as the probability of fall has passed a threshold.

2. Method according to claim 1, **characterized in that** the normalized distance obtained for the accelerometric signals is obtained according to the Mahalanobis distance in the time domain namely in the space of the time dependent kinetic energy distribution as follow :

$$d(S_t, S_{ref}) = e^{-(S_t - S_{ref})^T \theta^{-1}(S_t - S_{ref})}$$

in which $S_t$ is said signature at an instant t,
$S_{ref}$ is the normalized reference signature,
θ is a diagonal matrix containing the variances of the signatures of the representative sample of falls, taken over time.

3. Method according to claim 2, **characterized in that** the normalized reference signature $S_{ref}$ is obtained of a representative sample of falls as follows :

- identifying a particular value of the signature of each sample of falls, so as to carry out an alignment,
- aligning said signatures of each sample on the basis of the particular value, so as to obtain a group of aligned fall signatures,
- obtaining a reference signature in term of means and variances at each instant t from the values of the aligned signatures,
- deducting the squared value of the acceleration due to gravity so as to remove its influence and obtaining the normalized reference signature $S_{ref}$.

4. Method according to claim 1, **characterized in that** the normalized distance obtained with said at least one cardiovascular signal is obtained according to the Mahalanobis distance as follow :

$$d\left(S_{t,cardio,}\ S_{ref,cardio}\right) = 1 - e^{-\left(S_{t,cardio,}-S_{ref,cardio}\right)^T \theta_{cardio,act}^{-1}\left(S_{t,cardio,}-S_{ref,cardio}\right)}$$

where $S_{t,cardio}$, is the instantaneous signature comprising the at least one cardiovascular parameter,
$S_{ref,cardio}$ is the reference signature comprising the mean over time of the at least one items of cardiovascular information,
$\theta_{cardio}$ is a diagonal matrix containing the variances of the at least one cardiovascular information, taken over time.

5. Method according to claim 4, wherein several cardiovascular parameters are obtained, **characterized in that** said weighting is able to handle independently each cardiovascular parameter, and **in that** said weighting is dynamically adjusted and determined in function of the reliability of the respective distance measurements according to a detected situation.

6. Method according to claim 1, **characterized in that** it comprises, furthermore, a step of determining using cardiovascular parameters and/or of the accelerometric signals obtained via photoplethysmographic and/or accelerometric signals, whether the device is or is not being worn and possibly warn the user or, directly, alert a call centre which will then take the necessary measures and try to contact the person or trigger an intervention.

7. Method according to claim 1, **characterized in that** it comprises, furthermore, a step of determining using cardiovascular signals obtained through photoplethysmography whether the sensor is being worn properly and trigger the following actions:

- a safety step enabling the cardiovascular signals to be disregarded if the sensor is not being worn properly,
- a reliability step decreasing the probability of fall in case of a not worn or badly worn assessment.

**Patentansprüche**

1. Verfahren zur Erkennung eines Sturzes einer Person, umfassend die nachfolgenden Schritte:

- Einholen einer Signatur $S_t$ zu einem Zeitpunkt t, die die in drei Dimensionen von einem in einer Vorrichtung am Handgelenk eines Nutzers angeordneten Beschleunigungssensor gemessene Verteilung der kinetischen Energie der Person über ein Zeitfenster $\Delta t$ darstellt;
- Normalisieren dieser Signatur mittels einer unendlichen Norm oder einer Norm zweiter Ordnung,
- Einholen einer normalisierten Ähnlichkeitsmessung aufgrund eines normalisierten Abstandes zwischen der normalisierten Signatur $S_t$ und der normalisierten Referenzsignatur $S_{ref}$, wobei die Ähnlichkeitsmessung Proben von $S_{ref}$ und $S_t$ in Abhängigkeit von ihrer Zuverlässigkeit gewichtet, wobei die normalisierte Referenzsignatur $S_{ref}$ einer repräsentativen Sturzprobe entnommen wird,
- Einholen mindestens eines kardiovaskulären Parameters der Person über einen in der Vorrichtung angeordneten fotoplethysmographischen Sensor und Erzeugen fotoplethysmographischer Signale,

- Erhöhen der Qualität der fotoplethysmographischen Signale mithilfe der Daten des Beschleunigungssensors zur Sturzerkennung,

- Einholen einer Information über den Aktivitätsgrad der Person vom Beschleunigungssensor und einer Referenzsignatur $S_{ref,cardio}$ des kardiovaskulären Parameters aus den fotoplethysmographischen Signalen für einen gegebenen Aktivitätsgrad,

- Aufstellen eines kardiovaskulären Vergleichs mithilfe von Unähnlichkeitsmessungen auf der Grundlage eines normalisierten Abstandes zwischen einer momentanen Signatur $S_{t,cardio}$ und der Referenzsignatur $S_{ref,cardio}$ und

- Kombinieren der Ergebnisse des Beschleunigungssensors und der kardiovaskulären Vergleiche durch Summieren des Abstandes aus den fotoplethysmographischen Signalen mit denen der Beschleunigungssignale mit entsprechender Gewichtung,

- Verwenden des Kombinationsergebnisses als Indikator für die Wahrscheinlichkeit eines Sturzes und

- Ggf. Auslösen eines Warnsignals, wenn die Wahrscheinlichkeit eines Sturzes eine bestimmte Schwelle überschritten hat.

2. Verfahren nach Anspruch 1, **dadurch gekennzeichnet, dass** der sich aus den Beschleunigungssignalen ergebende normalisierte Abstand sich aus dem Mahalanobis-Abstand in der Zeitdomäne, d.h. im Bereich der zeitabhängigen kinetischen Energieverteilung, ergibt:

$$d(S_t, S_{ref}) = e^{-(S_t - S_{ref})^T \theta^{-1}(S_t - S_{ref})}$$

wobei $S_t$ die Signatur zu einem Zeitpunkt t ist,
$S_{ref}$ die normalisierte Referenzsignatur ist,
$\theta$ eine diagonale Matrix ist, die die Varianzen der Signaturen der repräsentativen Sturzprobe über die Zeit enthält.

3. Verfahren nach Anspruch 2, **dadurch gekennzeichnet, dass** die normalisierte Referenzsignatur $S_{ref}$ nach dem nachfolgenden Verfahren einer repräsentativen Sturzprobe entnommen wird:

- Identifizieren eines bestimmten Werts der Signatur jeder Sturzprobe, um eine Ausrichtung vorzunehmen,

- Ausrichten der Signaturen jeder Probe aufgrund des bestimmten Werts, um eine Gruppe miteinander ausgerichteter Sturzsignaturen herzustellen,

- Einholen einer Referenzsignatur im Sinne von Mitteln und Varianzen zu jedem Zeitpunkt t aus den Werten der ausgerichteten Signaturen,

- Abziehen des Quadrats der schwerkraftbedingten Beschleunigung, um deren Einfluss zu entfernen, und Einholen der normalisierten Referenzsignatur $S_{ref}$.

4. Verfahren nach Anspruch 1, **dadurch gekennzeichnet, dass** der sich aus dem mindestens einen kardiovaskulären Signal ergebende normalisierte Abstand nach dem nachfolgenden Verfahren aufgrund des Mahalanobis-Abstandes ermittelt wird:

$$d\left(S_{t,cardio} \quad S_{ref,cardio}\right) = 1 - e^{-\left(S_{t,cardio} - S_{ref,cardio}\right)^T \theta_{cardio}^{-1}\left(S_{t,cardio} - S_{ref,cardio}\right)}$$

wobei $S_{t,cardio}$ die momentane Signatur ist, die den mindestens einen kardiovaskulären Parameter umfasst,
$S_{ref,cardio}$ die Referenzsignatur ist, die das Mittel der mindestens einen kardiovaskulären Information über die Zeit umfasst,
$\theta_{cardio}$ eine diagonale Matrix ist, die die Varianzen der mindestens einen kardiovaskulären Information über die Zeit enthält.

5. Verfahren nach Anspruch 4, wobei mehrere kardiovaskuläre Parameter ermittelt werden, **dadurch gekennzeichnet, dass** die Gewichtung auf jeden kardiovaskulären Parameter unabhängig anwendbar ist, und dass die Gewichtung dynamisch eingestellt ist und sich nach der Zuverlässigkeit der jeweiligen Abstandsmessungen nach einer erkannten Situation bestimmt.

**6.** Verfahren nach Anspruch 1, **dadurch gekennzeichnet, dass** es ferner einen Schritt umfasst, in dem anhand der kardiovaskulären Parameter und/oder der Beschleunigungssignale aus den fotoplethysmographischen und/oder Beschleunigungssignalen bestimmt wird, ob die Vorrichtung getragen wird oder nicht und der Benutzer zu warnen ist oder ein Callcenter direkt zu benachrichtigen ist, das dann die erforderlichen Maßnahmen ergreift und versucht, mit der Person Kontakt aufzunehmen oder einen Eingriff auszulösen.

**7.** Verfahren nach Anspruch 1, **dadurch gekennzeichnet, dass** es ferner einen Schritt umfasst, in dem anhand der kardiovaskulären Signale aus der Fotoplethysmographie ermittelt wird, ob der Sensor richtig getragen wird, und die nachfolgenden Handlungen ausgelöst werden:

- ein Sicherheitsschritt, in dem die kardiovaskulären Signale außer Acht gelassen werden können, wenn der Sensor nicht richtig getragen wird,
- ein Zuverlässigkeitsschritt zur Senkung der Wahrscheinlichkeit eines Sturzes im Falle einer nicht oder falsch getragenen Vorrichtung.

**Revendications**

**1.** Procédé de détection d'une chute d'une personne, comprenant les étapes suivantes :

- obtention d'une signature $S_t$ à un instant t, représentant la distribution sur une fenêtre de temps $\Delta t$ de l'énergie cinétique de la personne, mesurée en trois dimensions par un capteur accélérométrique placé dans un dispositif situé sur le poignet d'un utilisateur,
- normalisation de ladite signature par une norme infinie ou du second ordre,
- obtention d'une mesure de similarité normalisée sur la base d'une distance normalisée entre la signature normalisée $S_t$ et la signature de référence normalisée $S_{ref}$, ladite mesure de similarité pondérant les échantillons de $S_{ref}$ et $S_t$ en fonction de leur fiabilité, où la signature de référence normalisée $S_{ref}$ a été obtenue à partir d'un échantillon représentatif de chutes,
- obtention d'au moins un paramètre cardiovasculaire de la personne par l'intermédiaire d'un capteur pléthysmographique optique placé dans ledit dispositif et fournissant des signaux pléthysmographiques optiques,
- utilisation des informations provenant du capteur accélérométrique utilisé pour détecter la chute, pour améliorer la qualité des signaux pléthysmographiques optiques,
- obtention, à partir du capteur accélérométrique, d'un élément d'informations concernant le niveau d'activité de la personne et obtention, à partir des signaux pléthysmographiques optiques d'une signature de référence $S_{ref,cardio}$ du paramètre cardiovasculaire pour un niveau d'activité donné,
- obtention d'une comparaison au niveau cardiovasculaire au moyen de mesures de différence, basée sur une distance normalisée entre une signature instantanée $S_{t,cardio}$ et la signature de référence $S_{ref,cardio}$, et
- combinaison des résultats de l'accéléromètre et des comparaisons au niveau cardiovasculaire, en sommant avec une pondération appropriée la distance obtenue avec les signaux pléthysmographiques optiques et celle obtenue avec les signaux accélérométriques,
- utilisation du résultat de la combinaison en tant qu'indicateur de probabilité de chute, et
- éventuellement, déclenchement d'un signal d'avertissement lorsque la probabilité de chute a dépassé un seuil.

**2.** Procédé selon la revendication 1, **caractérisé en ce que** la distance normalisée obtenue pour les signaux accélérométriques est obtenue en fonction de la distance de Mahalanobis dans le domaine temporel, à savoir dans l'espace de la distribution de l'énergie cinétique en fonction du temps comme suit :

$$d(S_t, S_{ref}) = e^{-(S_t - S_{ref})^T \theta^{-1}(S_t - S_{ref})}$$

où $S_t$ est ladite signature à un instant t,
$S_{ref}$ est la signature de référence normalisée,
$\theta$ est une matrice diagonale contenant les variances des signatures de l'échantillon représentatif de chutes, prises dans le temps.

**3.** Procédé selon la revendication 2, **caractérisé en ce que** la signature de référence normalisée $S_{ref}$ est obtenue à partir d'un échantillon représentatif de chutes en :

- identifiant une valeur particulière de la signature de chaque échantillon de chutes, de manière à effectuer un alignement,
- alignant lesdites signatures de chaque échantillon sur la base de la valeur particulière, de manière à obtenir un groupe de signatures de chutes alignées,
- obtenant une signature de référence en termes de moyennes et de variances à chaque instant t à partir des valeurs des signatures alignées,
- déduisant la valeur élevée au carré de l'accélération due à la gravité de manière à retirer son influence et obtenant la signature de référence normalisée $S_{ref}$.

4. Procédé selon la revendication 1, **caractérisé en ce que** la distance normalisée obtenue avec ledit au moins un signal cardiovasculaire est obtenue en fonction de la distance de Mahalanobis comme suit :

$$d(S_{t,cardio}, S_{ref,cardio}) = 1 - e^{-(S_{t,cardio}-S_{ref,cardio})^T \theta^{-1}_{cardio,act}(S_{t,cardio}-S_{ref,cardio})}$$

où $S_{t,cardio}$ est la signature instantanée comprenant le au moins un paramètre cardiovasculaire,
$S_{ref,cardio}$ est la signature de référence comprenant la moyenne dans le temps du au moins un élément d'information cardiovasculaire,
$\theta_{cardio}$ est une matrice diagonale contenant les variances de la au moins une information cardiovasculaire, prise dans le temps.

5. Procédé selon la revendication 4, dans lequel plusieurs paramètres cardiovasculaires sont obtenus, **caractérisé en ce que** ladite pondération est capable de gérer de manière indépendante chaque paramètre cardiovasculaire, et **en ce que** ladite pondération est ajustée dynamiquement et déterminée en fonction de la fiabilité des mesures de distance respectives en fonction d'une situation détectée.

6. Procédé selon la revendication 1, **caractérisé en ce qu'**il comprend, en outre, une étape de détermination, en utilisant des paramètres cardiovasculaires et/ou des signaux accélérométriques obtenus par l'intermédiaire de signaux pléthysmographiques optiques et accélérométriques, si le dispositif est ou n'est pas porté et éventuellement s'il convient d'avertir l'utilisateur ou, directement, d'alerter un centre d'appels qui prendra ensuite les mesures nécessaires et tentera de contacter la personne ou de déclencher une intervention.

7. Procédé selon la revendication 1, **caractérisé en ce qu'**il comprend, en outre, une étape de détermination, en utilisant des signaux cardiovasculaires obtenus par pléthysmographie optique, si le capteur est porté correctement et s'il convient de déclencher les actions suivantes :

- une étape de sécurité permettant que les signaux cardiovasculaires soient ignorés si le capteur n'est pas porté correctement,
- une étape de fiabilité diminuant la probabilité de chute dans le cas où le capteur n'est pas porté ou est mal porté.

Fig. 1

Fig. 2a

Fig. 2b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2829862

- US 20030153836 A

**Non-patent literature cited in the description**

- **J.R. DELLER ; J.G. PROAKIS ; J.H.L. HANSEN.** Discrete-Time Processing of Speech Signals. Prentice Hall, 1987

- **T. DEGEN ; H. JAECKAL ; M. RUFER ; S. WYSS.** SPEEDY: a fall detector in a wristwatch. *Proc. On Wearable Computers,* 2005, 184-187